# EUROPÄISCHE PATENTSCHRIFT

(11) **EP 1 134 271 B1**
(45) Veröffentlichungstag und Bekanntmachung des Hinweises auf die Patenterteilung: **19.05.2010**
(21) Anmeldenummer: 01105437.6
(22) Anmeldetag: 13.03.2001
(51) Int. Cl.: C10G 70/00, C07C 7/163, C07C 11/06

(54) **Verfahren zur flexiblen Herstellung von Propen und Hexen**
Process for producing propene and hexene
Procédé pour la production de propylène et de hexène

(30) Priorität: 17.03.2000 DE 10013253
(43) Veröffentlichungstag der Anmeldung: 19.09.2001
(73) Patentinhaber: BASF SE, 67056 Ludwigshafen (DE)
(72) Erfinder: Schwab, Peter, Dr., 67098 Bad Dürkheim (DE); Schulz, Ralf, Dr., 67346 Speyer (DE); Huber, Sylvia, Dr., 64673 Zwingenberg (DE)

(56) Entgegenhaltungen:
- EP-A- 0 742 195
- DE-A- 19 813 720
- DE-A- 19 932 060

## Beschreibung

Die vorliegende Erfindung betrifft ein Verfahren zur Umwandlung von olefinischen C₄-Kohlenwasserstoffen, beispielsweise aus Steamcrackern oder FCC-Crackern, in Propen und Hexen mittels Metathesereaktion.

Steamcracker stellen die Hauptquelle für petrochemische Basischemikalien, wie Ethen, Propen, C₄-Olefine und höhere Kohlenwasserstoffe dar. Beim Crackprozeß ist es notwendig, große Energiemengen bei hohen Temperaturen in einer Zeitspanne zu übertragen, die einerseits zwar ausreicht, um die Spaltung durchzuführen, andererseits aber eine Weiterreaktion der Spaltprodukte nicht zuläßt. Bei der Spaltung von Kohlenwasserstoffen wird die Ausbeute an Ethen und Propen daher im wesentlichen durch die
- Art der eingesetzten Kohlenwasserstoffe (Naphtha, Ethan, LPG, Gasöl, o.ä.)
- Spalttemperatur,
- Verweilzeit
- und die Partialdrücke der jeweiligen Kohlenwasserstoffe
bestimmt.

Die höchste Ausbeute an Ethen und Propen wird bei Spalttemperaturen zwischen 800 und 850°C und Verweilzeiten von 0,2 bis 0,5 s erzielt. Hauptprodukt ist in diesem Bereich stets Ethen, wobei das C₃/C₂-Austragsverhältnis von ca. 0,5 bis 0,7 durch Variation der CrackBedingungen in geringem Ausmaß erhöht werden kann. Weltweit steigt der Bedarf an Propen rascher an als der von Ethen. Dies hat u.a. zur Folge, daß Verfahren zur Downstream-Verwertung der höheren, beim Crackprozeß gebildeten Kohlenwasserstoffe, wie C₄, in Hinblick auf die Optimierung der Propenausbeute stark an Bedeutung gewinnen. Ein geeignetes Verfahren ist die Olefinmetathese.

Die Olefinmetathese (Disproportionierung) beschreibt in ihrer einfachsten Form die reversible, metallkatalysierte Umalkylidenierung von Olefinen durch Bruch oder Neuformierung von C-C-Doppelbindungen gemäß nachfolgender Gleichung:

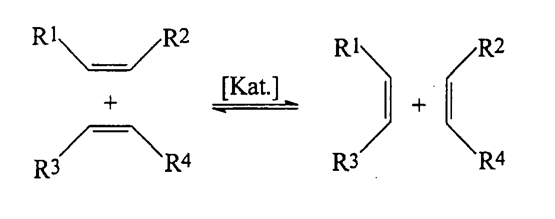

Im speziellen Fall der Metathese von acyclischen Olefinen unterscheidet man zwischen Selbstmetathese, bei der ein Olefin in ein Gemisch zweier Olefine unterschiedlicher molarer Masse übergeht (beispielsweise: Propen → Ethen + 2-Buten), und Kreuz- oder Co-Metathese, die eine Reaktion zweier unterschiedlicher Olefine beschreibt (Propen + 1-Buten → Ethen + 2-Penten). Ist einer der Reaktionspartner Ethen, so spricht man im allgemeinen von einer Ethenolyse.

Als Metathesekatalysatoren eignen sich prinzipiell homogene und heterogene Übergangsmetall-Verbindungen, insbesondere die der VI. bis VIII.-Nebengruppe des Periodensystems der Elemente, sowie homogene und heterogene Katalysatorsysteme, in denen diese Verbindungen enthalten sind.

Unterschiedliche Metathese-Verfahren, die von C₄-Strömen ausgehen, sind beschrieben.

US 5,057,638 betrifft ein Verfahren zur Herstellung von 1-Hexen, umfassend die Verfahrensschritte:
a) Metathese von 1-Buten zu einer Mischung aus 3-Hexen und Ethen,
b) Abtrennen des 3-Hexens aus dem in Schritt a) gewonnenen Produktgemisch
c) Umsetzung des 3-Hexens mit einem reaktiven Wasserstoff, vorzugsweise aus Wasser oder Carbonsäure stammend, enthaltenden Elektrophil unter sauren Bedingungen, welche die Addition der elektrophilen Komponenten an die C=C-Bindung erlauben (z.B. Hydratisierung),
d) Cracken des Produkts aus Schritt c), z.B. durch Dehydratisierung, zur Herstellung eines Gemisches aus n-Hexenen, welches 1-Hexen in wirtschaftlich akzeptablen Mengen enthält.

EP-A-0 742 195 betrifft ein Verfahren zur Umwandlung von C₄- oder C₅-Schnitten in Ether und Propylen. Ausgehend von C₄-Schnitten werden zunächst enthaltene Diolefine und acetylenische Verunreinigungen selektiv hydriert, wobei die Hydrierung mit einer Isomerisierung von 1-Buten zu 2-Buten verbunden ist. Die Ausbeute an 2-Butenen soll dabei maximiert werden. Das Verhältnis von 2-Buten zu 1-Buten beträgt nach der Hydrierung etwa 9:1. Es schließt sich eine Veretherung der enthaltenen Isooelfine an, wobei die Ether aus dem C₄-Schnitt abgetrennt werden. Sodann werden Oxigenat-Verunreinigungen abgetrennt. Der erhaltene, neben Alkanen überwiegend 2-Buten enthaltende Austrag wird sodann mit Ethylen in Gegenwart eines Metathesekatalysators umgesetzt, um einen Reaktionsaustrag zu erhalten, der Propylen als Produkt enthält. Die Metathese wird in Gegenwart eines Katalysators, der Rheniumoxid auf einem Träger enthält, durchgeführt.

DE-A-198 13 720 betrifft ein Verfahren zur Herstellung von Propen aus einem C₄-Strom. Dabei werden zunächst Butadien und Isobuten aus dem C₄-Strom entfernt. Sodann werden Oxigenat-Verunreinigungen abgetrennt, und es wird eine zweistufige Metathese der Butene durchgeführt. Zunächst werden 1-Buten und 2-Buten zu Propylen und 2-Penten umgesetzt. Sodann wird das erhaltene 2-Penten mit zudosiertem Ethylen zu Propylen und 1-Buten weiter umgesetzt.

Die prioritätsältere, nicht vorveröffentlichte DE-A-199 32 060 betrifft ein Verfahren zur Herstellung von C₅-/C₆-Olefinen durch Umsetzung eines Ausgangsstroms, der 1-Buten, 2-Buten und Isobuten enthält, zu einem Gemisch aus C₂₋₆-Olefinen, Dabei wird aus Butenen insbesondere Propen gewonnen. Zusätzlich werden Hexen und Methylpenten als Produkte ausgeschleust. In der Metathese wird kein Ethen zudosiert. Gegebenenfalls wird in der Metathese gebildetes Ethen in den Reaktor zurückgeführt.

Die Weltmarktpreise von Ethen und Propen sind Veränderungen unterworfen. Zudem besteht eine Nachfrage an Penten- und Hexen-Olefinfraktionen, die als kostengünstige alternative Rohstoffe für Weichmacher- oder Tensidalkohole eingesetzt werden können. Um auf die Preisänderungen im Weltmarkt reagieren zu können, besteht Nachfrage nach einem Verfahren zur flexiblen Herstellung von Propen und Hexen, das es je nach Preisdifferenz zwischen Ethen und Propen erlaubt, das erhaltene Wertstoffspektrum entsprechend anzupassen. Beispielsweise soll es möglich sein, Penten- und Hexen-Olefinfraktionen sowie Propen unter Einsatz geringer bis großer Mengen Ethen flexibel zu erhalten.

Aufgabe der vorliegenden Erfindung ist die Bereitstellung eines flexibel steuerbaren katalytischen Verfahrens zur Gewinnung von Propen und Hexen aus kostengünstig verfügbaren olefinhaltigen C₄-Kohlenwasserstoffgemischen. Dabei soll eine möglichst flexible Ethenzudosierung möglich sein, mit der die jeweiligen Mengen der erhaltenen Produkte, insbesondere Propen und Hexen, beeinflußt werden können. Damit soll die Wertschöpfung von Steamcracker-Nebenprodukten verbessert werden, wobei die Produkte mit der größten Wertschöpfung erhalten werden können.

Die Aufgabe wird erfindungsgemäß gelöst durch ein Verfahren zur Herstellung von Propen und Hexen aus einem olefinische C₄-Kohlenwasserstoffe enthaltenden Raffinat-II-Ausgangsstrom, bei dem
a) in Gegenwart eines Metathesekatalysators, der mindestens eine Verbindung eines Metalls der VI.b, VII.b oder VIII. Nebengruppe des Periodensystems der Elemente enthält, eine Metathesereaktion durchgeführt wird, im Rahmen derer im Ausgangsstrom enthaltene Butene mit Ethen zu einem Ethen, Propen, Butene, 2-Penten, 3-Hexen und Butane enthaltenden Gemisch umgesetzt werden, wobei bezogen auf die Butene 0,05 bis 0,6 Moläquivalente Ethen eingesetzt werden,
b) der so erhaltene Austragsstrom zunächst destillativ getrennt wird in eine C₂-C₃-Olefine enthaltende Leichtsiederfraktion **A** sowie in eine C₄-C₆-Olefine und Butane enthaltende Schwersiederfraktion,
c) die aus b) erhaltene Leichtsiederfraktion A anschließend destillativ in eine Ethen enthaltende Fraktion und eine Propen enthaltende Fraktion getrennt wird, wobei die Ethen enthaltende Fraktion in den Verfahrensschritt a) zurückgeführt wird und die Propen enthaltende Fraktion als Produkt ausgeschleust wird,
d) die aus b) erhaltene Schwersiederfraktion anschließend destillativ in eine Butene und Butane enthaltende Leichtsiederfraktion **B**, eine Penten enthaltende Mittelsiederfraktion **C** und in eine Hexen enthaltende Schwersiederfraktion **D** getrennt wird,
e) wobei die Fraktionen **B** und **C** komplett oder teilweise in den Verfahrensschritt a) zurückgeführt werden und die Fraktion **D** als Produkt ausgeschleust wird.

Die einzelnen Ströme und Fraktionen können die genannten Verbindungen enthalten oder aus ihnen bestehen. Im Fall, daß sie aus den Strömen oder Verbindungen bestehen, ist die Gegenwart kleinerer Mengen anderer Kohlenwasserstoffe nicht ausgeschlossen.

Dabei wird in einstufiger Reaktionsführung in einer Metathesereaktion eine aus C₄-Olefinen, vorzugsweise n-Butenen und Butanen bestehende Fraktion mit variablen Mengen Ethen an einem homogenen oder vorzugsweise heterogenen Metathesekatalysator zu einem Produktgemisch aus (inerten) Butanen, nicht umgesetztem 1-Buten, 2-Buten sowie den Metatheseprodukten Ethen, Propen, 2-Penten und 3-Hexen umgesetzt. Die gewünschten Produkte Propen und 3-Hexen werden ausgeschleust, und die verbleibenden Produkte und nicht umgesetzten Verbindungen werden in die Metathese ganz oder teilweise zurückgeführt. Vorzugsweise werden sie möglichst vollständig zurückgeführt, wobei nur geringe Mengen ausgeschleust werden, um eine Aufpegelung zu vermeiden. Idealerweise kommt es zu keiner Aufpegelung, und alle Verbindungen außer 3-Hexen und Propen werden in die Metathese zurückgeführt.

Erfindungsgemäß werden, bezogen auf die Butene im C₄-Feedstrom, 0,05 bis 0,6, vorzugsweise 0,1 bis 0,5 Moläquivalente Ethen eingesetzt. Damit werden im Vergleich zum Stand der Technik nur geringe Ethenmengen eingesetzt. Beispielsweise werden gemäß EP-A-0 742 195 mindestens äquivalente molare Mengen an Ethen, bezogen auf die Butene, eingesetzt. Vorzugsweise wird mit einem Ethenüberschuß gearbeitet.

Wenn auf die Zuführung von zusätzlichem Ethen verzichtet würde, würden im Verfahren nur bis zu maximal etwa 1,5 %, bezogen auf die Umsetzungsprodukte, an Ethen gebildet, das zurückgeführt wird. Im Unterschied zur prioritätsälteren, nicht vorveröffentlichten DE-A-199 32 060 werden erfindungsgemäß größere Ethenmengen eingesetzt, wobei die eingesetzten Mengen wesentlich geringer sind als in den bekannten Verfahren zur Herstellung von Propen.

Zudem werden erfindungsgemäß maximal mögliche Mengen an im Reaktoraustrag enthaltenen C₄-Produkten und C₅-Produkten zurückgeführt. Dies betrifft insbesondere die Rückführung von nicht umgesetztem 1-Buten und 2-Buten sowie gebildetem 2-Penten.

Sofern im C₄-Feedstrom noch geringe Mengen an Isobuten enthalten sind, können auch geringe Mengen verzweigter Kohlenwasserstoffe gebildet werden.

Die Menge an möglicherweise zusätzlich gebildeten verzweigten C₅- und C₆-Kohlenwasserstoffen im Metatheseaustrag ist abhängig vom Isobuten-Gehalt im C₄-Feed und wird vorzugsweise möglichst gering (< 3 %) gehalten.

Um das erfindungsgemäße Verfahren in mehreren Variationen näher zu erläutern, wird die im Metathesereaktor stattfindende Umsetzung in drei wichtige Einzelreaktionen unterteilt:
**1. Kreuzmetathese von 1-Buten mit 2-Buten** 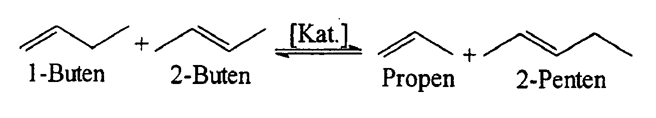
**2. Selbstmetathese von 1-Buten** 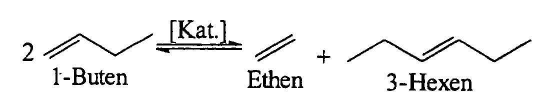
**3. Ethenolyse von 2-Buten**

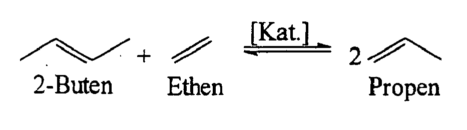

In Abhängigkeit vom jeweiligen Bedarf an den Zielprodukten Propen und 3-Hexen (die Bezeichnung 3-Hexen beinhaltet unter anderem eventuell gebildete Isomere) kann die äußere Massenbilanz des Verfahrens gezielt durch variablen Einsatz von Ethen und durch Verschiebung des Gleichgewichts durch Rückführung bestimmter Teilströme beeinflußt werden. So wird beispielsweise die 3-Hexenausbeute dadurch erhöht, daß durch Rückführung von 2-Penten in den Metatheseschritt die Kreuzmetathese von 1-Buten mit 2-Buten unterdrückt wird, so daß hier kein oder möglichst wenig 1-Buten verbraucht wird. Bei der dann bevorzugt ablaufenden Selbstmetathese von 1-Buten zu 3-Hexen wird zusätzlich Ethen gebildet, welches in einer Folgereaktion mit 2-Buten zum Wertprodukt Propen reagiert.

Olefingemische, die 1-Buten und 2-Buten und gegebenenfalls Isobuten enthalten, werden u.a. bei diversen Crackprozessen wie Steamcracking oder FCC-Cracking als C₄-Fraktion erhalten. Alternativ können Butengemische, wie sie bei der Dehydrierung von Butanen oder durch Dimerisierung von Ethen anfallen, eingesetzt werden. In der C₄-Fraktion enthaltene Butane verhalten sich inert. Diene, Alkine oder Enine werden vor dem erfindungsgemäßen Metatheseschritt mit gängigen Methoden wie Extraktion oder Selektivhydrierung entfernt.

Der Butengehalt der im Verfahren eingesetzten C₄-Fraktion beträgt 1 bis 100 Gew.-%, vorzugsweise 60 bis 90 Gew.-%. Der Butengehalt bezieht sich dabei auf 1-Buten, 2-Buten und Isobuten.

Vorzugsweise wird eine C₄-Fraktion eingesetzt, wie sie beim Steam- oder FCC-Cracken oder bei der Dehydrierung von Butan anfällt.

Dabei wird als C₄-Fraktion vorzugsweise Raffinat II eingesetzt, wobei der C₄-Strom vor der Metathese-Reaktion durch entsprechende Behandlung an Adsorber-Schutzbetten, bevorzugt an hochoberflächigen Aluminiumoxiden oder Molsieben von störenden Verunreinigungen befreit wird.

Die aus Schritt b) erhaltene Leichtsiederfraktion A, die C₂-C₃-Olefine enthält, wird destillativ in eine Ethen enthaltende Fraktion und eine Propen enthaltende Fraktion getrennt. Die Ethen enthaltende Fraktion wird sodann in den Verfahrensschritt a), d.h. die Metathese, zurückgeführt, und die Propen enthaltende Fraktion wird als Produkt ausgeschleust.

In Schritt d) kann die Trennung in Leichtsiederfraktion B, Mittelsiederfraktion C und Schwersiederfraktion D beispielsweise in einer Trennwandkolonne durchgeführt werden. Hierbei wird die Leichtsiederfraktion B über Kopf, die Mittelsiederfraktion C über einen Mittelaustrag und die Schwersiederfraktion D als Sumpf erhalten.

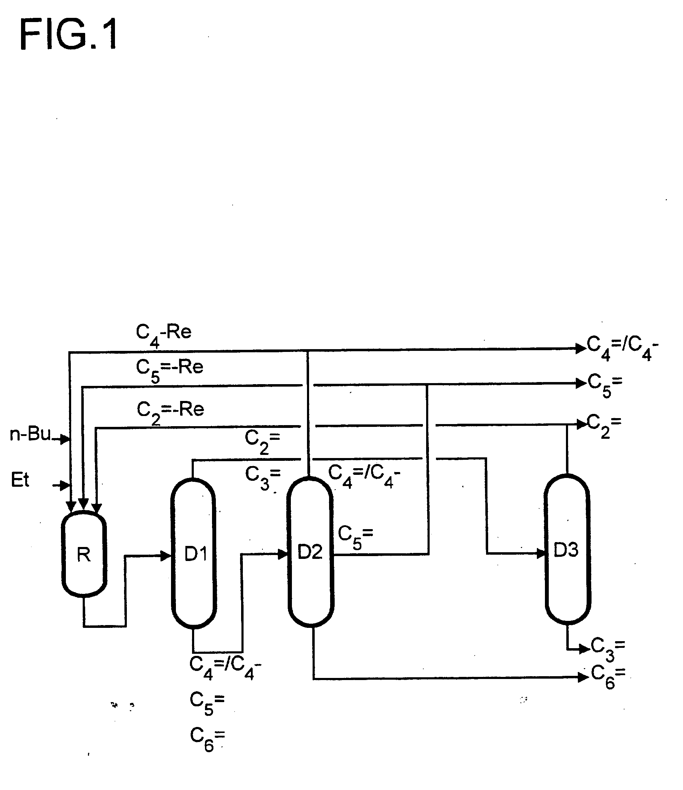

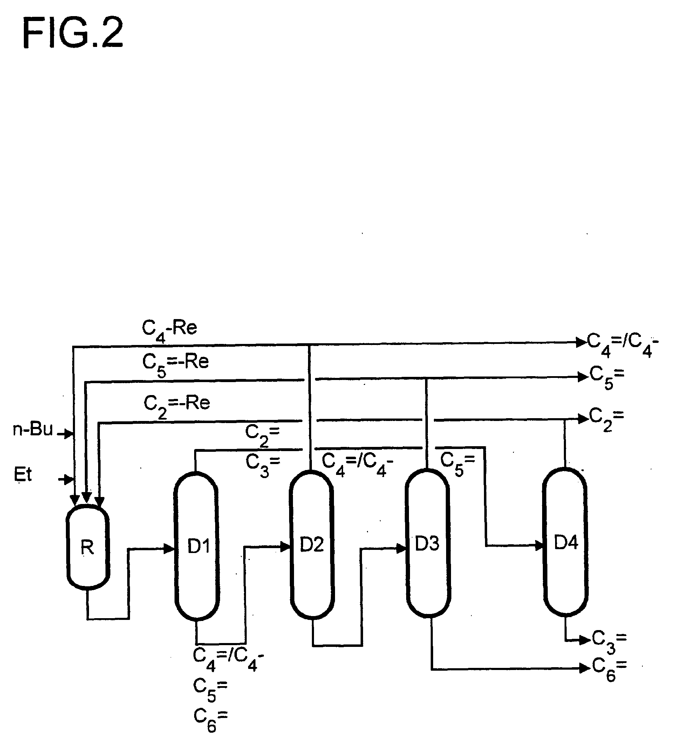

Um die bei dem flexibel gesteuerten Verfahren anfallenden unterschiedlich großen Mengen an Produkten besser handhaben zu können, ist es jedoch vorteilhaft, eine zweistufige Auftrennung der aus b) erhaltenen Schwersiederfraktion durchzuführen. Vorzugsweise wird die b) erhaltene Schwersiederfraktion zunächst destillativ in eine Butene und Butane enthaltende Leichtsiederfraktion B und eine 2-Penten und 3-Hexen enthaltende Hochsiederfraktion getrennt. Die Hochsiederfraktion wird sodann destillativ in die Fraktionen C und D getrennt. Die beiden Ausführungsformen sind in den Abbildungen 1 und 2 näher erläutert.

Die Metathesereaktion wird dabei vorzugsweise in Gegenwart von heterogenen, nicht oder nur geringfügig isomerisierungsaktiven Metathesekatalysatoren durchgeführt, die aus der Klasse der auf anorganischen Trägern aufgebrachten Übergangsmetallverbindungen von Metallen der VI.b, VII.b oder VIII.-Gruppe des Periodensystems der Elemente ausgewählt sind.

Bevorzugt wird als Metathesekatalysator Rheniumoxid auf einem Träger, vorzugsweise auf γ-Aluminiumoxid oder auf Al₂O₃/B₂O₃/SiO₂-Mischträgern eingesetzt.

Insbesondere wird als Katalysator Re₂O₇/γ-Al₂O₃ mit einem Rheniumoxid-Gehalt von 1 bis 20 %, vorzugsweise 3 bis 15 %, besonders bevorzugt 6 bis 12 % (Gew.-%) eingesetzt.

Die Metathese wird bei Flüssigfahrweise vorzugsweise bei einer Temperatur von 0 bis 150°C, besonders bevorzugt 20 bis 80°C sowie einem Druck von 2 bis 200 bar, besonders bevorzugt 5 bis 30 bar, durchgeführt.

Wenn die Metathese in der Gasphase durchgeführt wird, beträgt die Temperatur vorzugsweise 20 bis 300°C, besonders bevorzugt 50 bis 200°C. Der Druck beträgt in diesem Fall vorzugsweise 1 bis 20 bar, besonders bevorzugt 1 bis 5 bar.

Im Rahmen von Arbeiten zur Verbesserung der Wertschöpfung von Steamcracker-Nebenprodukten stellte sich zudem die Aufgabe, eine flexibel steuerbare Verfahrenssequenz zur Verwertung von C₄-Schnitt zu entwickeln. Ziel war es, C₄-Olefine mit hoher Wertschöpfung in höherpreisige Olefinfraktionen umzuwandeln. Als Einsatzstoff steht Roh-C₄-Schnitt aus Steamcrackern oder FCC-Crackern zur Verfügung.

Die Aufgabe wird erfindungsgemäß gelöst durch ein Verfahren zur Herstellung von C₅/C₆-Olefinen und Propen aus Steamcracker- oder Raffinerie-C₄-Strömen, umfassend die Teilschritte
(1) Abtrennung von Butadien und acetylenischen Verbindungen durch gegebenenfalls Extraktion von Butadien mit einem Butadien-selektiven Lösungsmittel und nachfolgend /oder Selektivhydrierung von in Roh-C₄-Schnitt enthaltenen Butadienen und acetylenischen Verunreinigungen um einen Reaktionsaustrag zu erhalten, der n-Butene und Isobuten und im wesentlichen keine Butadiene und acetylenischen Verbindungen enthält,
(2) Abtrennung von Isobuten durch Umsetzung des in der vorstehenden Stufe erhaltenen Reaktionsaustrags mit einem Alkohol in Gegenwart eines sauren Katalysators zu einem Ether, Abtrennung des Ethers und des Alkohols, die gleichzeitig oder nach der Veretherung erfolgen kann, um einen Reaktionsaustrag zu erhalten, der n-Butene und gegebenenfalls Oxygenat-Verunreinigungen enthält, wobei gebildeter Ether ausgetragen oder zur Reingewinnung von Isobuten rückgespalten werden kann und dem Veretherungsschritt ein Destillationsschritt zur Abtrennung von Isobuten nachfolgen kann, wobei gegebenenfalls auch eingeschleuste C₃-, i-C₄- sowie C₅-Kohlenwasserstoffe destillativ im Rahmen der Aufarbeitung des Ethers abgetrennt werden können, oder Oligomerisierung oder Polymerisation von Isobuten aus dem in der vorstehenden Stufe erhaltenen Reaktionsaustrag in Gegenwart eines sauren Katalysators, dessen Säurestärke zur selektiven Abtrennung von Isobuten als Oligo- oder Polyisobuten geeignet ist, um einen Strom zu erhalten, der 0 bis 15 % Rest-Isobuten aufweist,
(3) Abtrennen der Oxygenat-Verunreinigungen des Austrags der vorstehenden Schritte an entsprechend ausgewählten Adsorbermaterialien,
(4) Metathesereaktion des so erhaltenen Raffinats II-Stromes wie beschrieben.

Vorzugsweise wird der Teilschritt Selektivhydrierung von in Roh-C₄-Schnitt enthaltenen Butadien und acetylenischen Verunreinigungen zweistufig durchgeführt durch Inkontaktbringen des Roh-C₄-Schnittes in flüssiger Phase mit einem Katalysator, der mindestens ein Metall, ausgewählt aus der Gruppe Nickel, Palladium und Platin, auf einem Träger enthält, vorzugsweise Palladium auf Aluminiumoxid, bei einer Temperatur von 20 bis 200°C, einem Druck von 1 bis 50 bar, einer Volumengeschwindigkeit von 0,5 bis 30 m³ Frischfeed pro m³ Katalysator pro Stunde und einem Verhältnis von Recycle zu Zustrom von 0 bis 30 mit einem Molverhältnis von Wasserstoff zu Diolefinen von 0,5 bis 50, um einen Reaktionsaustrag zu erhalten, in welchem neben Isobuten die n-Butene 1-Buten und 2-Buten in einem Molverhältnis von 2:1 bis 1:10, vorzugsweise von 2:1 bis 1:3, vorliegen und im wesentlichen keine Diolefine und acetylenischen Verbindungen enthalten sind. Für einen maximalen Hexenaustrag liegt vorzugsweise 1-Buten im Überschuß vor, für eine hohe Propenausbeute liegt vorzugsweise 2-Buten im Überschuß vor. Das bedeutet, daß das gesamte Molverhältnis im ersten Fall 2:1 bis 1:1 und im zweiten Fall 1:1 bis 1:3 betragen kann.

Vorzugsweise wird der Teilschritt Butadien-Extraktion aus Roh-C₄-Schnitt mit einem Butadien-selektiven Lösungsmittel durchgeführt, ausgewählt aus der Klasse polaraprotischer Lösungsmittel, wie Aceton, Furfural, Acetonitril, Dimethylacetamid, Dimethylformamid und N-Methylpyrrolidon, um einen Reaktionsaustrag zu erhalten, in welchem nach anschließend durchgeführter Selektivhydrierung/Isomerisierung die n-Butene 1-Buten und 2-Buten in einem Molverhältnis 2:1 bis 1:10, vorzugsweise von 2:1 bis 1:3, vorliegen.

Vorzugsweise wird der Teilschritt Isobuten-Veretherung mit Methanol oder Isobutanol, vorzugsweise Isobutanol in Gegenwart eines sauren Ionentauschers in einer dreistufigen Reaktorkaskade durchgeführt, in der geflutete Festbettkatalysatoren von oben nach unten durchströmt werden, wobei die Reaktor-Eingangstemperatur 0 bis 60°C, vorzugweise 10 bis 50°C, die Ausgangstemperatur 25 bis 85°C, vorzugsweise 35 bis 75°C, der Druck 2 bis 50 bar, vorzugsweise 3 bis 20 bar und das Verhältnis von Isobutanol zu Isobuten 0,8 bis 2,0, vorzugsweise 1,0 bis 1,5 betragen sowie der Gesamtumsatz dem Gleichgewichtsumsatz entspricht.

Vorzugsweise wird der Teilschritt Isobuten-Abtrennung durch Oligomerisierung oder Polymerisation von Isobuten ausgehend von dem Reaktionsaustrag, der nach den vorstehend beschriebenen Stufen Butadien-Extraktion und/oder Selektivhydrierung erhalten wird, in Gegenwart eines Katalysators durchgeführt, der ausgewählt ist aus der Klasse homogener und heterogener Broensted- oder Lewis-Säuren.

### Selektivhydrierung von Roh-C₄-Schnitt

Alkine, Alkinene und Alkadiene sind aufgrund ihrer Neigung zur Polymerisation oder ihrer ausgeprägten Neigung zur Komplexbildung an Übergangsmetallen in vielen technischen Synthesen unerwünschte Stoffe. Sie beeinträchtigen die bei diesen Reaktionen verwendeten Katalysatoren zum Teil sehr stark.

Der C₄-Strom eines Steamcrackers enthält einen hohen Anteil mehrfach ungesättigter Verbindungen wie 1,3-Butadien, 1-Butin (Ethylacetylen) und Butenin (Vinylacetylen). Je nach vorhandener Downstream-Verarbeitung werden die mehrfach ungesättigten Verbindungen entweder extrahiert (Butadien-Extraktion) oder selektiv hydriert. Im erstgenannten Fall beträgt der Restgehalt mehrfach ungesättigter Verbindungen typischerweise 0,05 bis 0,3 Gew.-%, im letztgenannten Fall typischerweise 0,1 bis 4,0 Gew.-%. Da die Restmengen an mehrfach ungesättigten Verbindungen ebenfalls bei der Weiterverarbeitung stören, ist eine weitere Anreicherung durch Selektivhydrierung auf Werte < 10 ppm erforderlich. Um einen möglichst hohen Wertproduktanteil an Butenen zu erhalten, ist die Überhydrierung zu Butanen so gering wie möglich zu halten.

Geeignete Hydrierungskatalysatoren sind beschrieben:
- J.P.Boitiaux, J.Cosyns, M.Derrien and G.Lèger, Hydrocarbon Processing, March 1985, p.51-59
   Beschreibung bimetallischer Katalysatoren für Selektivhydrierungen von C₂-, C₃-, C₄-, C₅- und C₅₊-Kohlenwasserstoffströmen. Besonders bimetallische Katalysatoren aus Gp.VIII und Gp.IB-Metallen zeigen im Vergleich zu reinen Pd-Trägerkatalysatoren Verbesserungen in der Selektivität.
- DE-A-2 059 978
   Selektive Hydrierung von ungesättigten Kohlenwasserstoffen in flüssiger Phase an einem Pd/Tonerde-Katalysator. Der Katalysator ist dadurch gekennzeichnet, daß der Tonerde-Träger mit BET 120 m²/g zunächst einer Wasserdampf-Behandlung bei 110-300°C unterzogen wird und anschließend bei 500-1200°C calziniert wird. Zuletzt wird die Pd-Verbindung aufgebracht und bei 300-600°C calziniert.
- EP-A-0 564 328 **und** EP-A-0 564 329
   Katalysator, welcher u.a. aus Pd und In oder Ga auf Träger besteht. Die Katalysatorkombination ermöglicht einen Einsatz ohne CO-Zusatz bei hoher Aktivität und Selektivität.
- EP-A-0 089 252
   Pd, Au-Trägerkatalysatoren.
   Die Katalysatorherstellung umfaßt folgende Schritte:
   - Tränkung eines mineralischen Trägers mit einer Pd-Verbindung
   - Calzinierung unter O₂-haltigem Gas
   - Behandlung mit einem Reduktionsmittel
   - Tränkung mit einer halogenierten Au-Verbindung
   - Behandlung mit einem Reduktionsmittel
   - Auswaschen des Halogens durch basische Verbindung
   - Calzinierung unter O₂-haltigem Gas.
- US 5,475,173
   Katalysator bestehend aus Pd und Ag und Alkalifluorid auf anorganischem Träger. Vorteile des Katalysators: Durch KF-Zusatz erhöhter Butadien-Umsatz und bessere Selektivität zu Butenen (d.h. geringere Überhydrierung zu n-Butan).
- EP-A-0 653 243
   Katalysator zeichnet sich dadurch aus, daß sich die Aktivkomponente überwiegend in den Meso- und Makroporen befindet. Der Katalysator zeichnet sich weiterhin durch ein großes Porenvolumen und niedriges Rüttelgewicht aus. So besitzt der Katalysator aus Beispiel 1 ein Rüttelgewicht von 383 g/l und ein Porenvolumen von 1,17 ml/g.
- EP-A-0 211381
   Katalysator aus Gp.VIII-Metall (vorzugsweise Pt) und mindestens ein Metall aus Pb, Sn oder Zn auf anorganischem Träger. Der bevorzugte Katalysator besteht aus Pt/ZnAl₂O₄. Durch die genannten Promotoren Pb, Sn und Zn wird die Selektivität des Pt-Kontaktes verbessert.
- EP-A-0 722 776
   Katalysator aus Pd und mindestens einem Alkalifluorid und optional Ag auf anorganischen Träger (Al₂O₃, TiO₂ und/oder ZrO₂). Die Katalysatorkombination ermöglicht eine Selektivhydrierung in Gegenwart von Schwefel-Verbindungen.
- EP-A-0 576 828
   Katalysator auf Basis von Edelmetall und/oder Edelmetaloxid auf Al₂O₃-Träger mit definiertem Röntgenbeugungsmuster. Der Träger besteht dabei aus n-Al₂O₃ und/oder γ-Al₂O₃. Der Katalysator besitzt aufgrund des speziellen Trägers eine hohe Anfangsselektivität und kann daher sofort zur selektiven Hydrierung ungesättigter Verbindungen eingesetzt werden.
- JP 01110594
   Pd-Trägerkatalysator
   Zusätzlich wird ein weiterer Elektronendonor eingesetzt. Dieser besteht entweder aus einem auf dem Katalysator abgeschiedenem Metall, wie beispielsweise Na, K, Ag, Cu, Ga, In, Cr, Mo oder La, oder einem Zusatz zum Kohlenwasserstoff-Einsatzstoff, wie beispielsweise Alkohol, Ether oder N-haltige Verbindungen. Durch die genannten Maßnahmen kann eine Herabsetzung der 1-Buten-Isomerisierung erreicht werden.
- DE-A-31 19 850
   Katalysator aus einem SiO₂- oder Al₂O₃-Träger mit 10 bis 200 m²/g bzw. ≤ 100 m²/g mit Pd und Ag als Aktivkomponente. Der Katalysator dient vornehmlich zur Hydrierung butadienarmer Kohlenwasserstoffströme.
- EP-A-0 780 155
   Katalysator aus Pd und einem Gp. IB-Metall auf einem Al₂O₃-Träger, wobei mindestens 80 % des Pd und 80 % des Gp. IB-Metalls in einer äußeren Schale zwischen r₁ (= Radius des Pellets) und 0,8- r₁ aufgebracht sind.

### Alternativ: Extraktion von Butadien aus Roh-C₄-Schnitt

Das bevorzugte Verfahren zur Butadien-Isolierung basiert auf dem physikalischen Prinzip der Extraktivdestillation. Durch Zusatz selektiver organischer Lösungsmittel wird die Flüchtigkeit spezieller Komponenten eines Gemisches, in diesem Fall Butadien, erniedrigt. Diese bleiben daher mit dem Lösungsmittel im Sumpf der Destillationskolonne, während die destillativ zuvor nicht abtrennbaren Begleitsubstanzen über Kopf entfernt werden können. Als Lösungsmittel für die Extraktivdestillation kommen hauptsächlich Aceton, Furfural, Acetonitril, Dimethylacetamid, Dimethylformamid (DMF) und N-Methylpyrrolidon (NMP) zur Anwendung. Extraktivdestillationen eignen sich besonders für butadienreiche C₄-Crackschnitte mit einem relativ hohen Anteil an Alkinen, u.a. Methyl-, Ethyl- und Vinylacetylen sowie Methylallen.

Das vereinfachte Prinzip einer Lösungsmittel-Extraktion aus Roh-C₄-Schnitt kann wie folgt dargestellt werden: Der vollständig verdampfte C₄-Schnitt wird einer Extraktionskolonne am unteren Ende zugeführt. Das Lösungsmittel (DMF, NMP) fließt von oben dem Gasgemisch entgegen und belädt sich auf dem Weg nach unten mit besserlöslichem Butadien und geringen Mengen an Butenen. Am unteren Ende der Extraktionskolonne wird ein Teil des gewonnenen Rein-Butadiens zugeführt, um die Butene weitestgehend auszutreiben. Die Butene verlassen die Trennsäule am Kopf. In einer als Ausgaser bezeichneten weiteren Kolonne wird das Butadien durch Auskochen vom Lösungsmittel befreit und anschließend reindestilliert.

Üblicherweise wird der Reaktionsaustrag einer Butadien-Extraktivdestillation in die zweite Stufe einer Selektivhydrierung eingespeist, um den Butadien-Restgehalt auf Werte von < 10 ppm zu reduzieren.

Der nach Abtrennung von Butadien verbleibende C₄-Strom wird als C₄-Raffinat oder Raffinat I bezeichnet und enthält in der Hauptsache die Komponenten Isobuten, 1-Buten, 2-Butene sowie n- und Isobutane.

### Abtrennung von Isobuten aus Raffinat I

Bei der weiteren Auftrennung des C₄-Stromes wird nachfolgend vorzugsweise Isobuten isoliert, da es sich durch seine Verzweigung und seine höhere Reaktivität von den übrigen C₄-Komponenten unterscheidet. Neben der Möglichkeit einer formselektiven Molsiebtrennung, mit welcher Isobuten mit einer Reinheit von 99 % gewonnen werden kann und an den Molsiebporen adsorbierte n-Butene und Butan mittels eines höhersiedenden Kohlenwasserstoffs wieder desorbiert werden können, geschieht dies in erster Linie destillativ unter Verwendung eines sog. Deisobutenizers, mit welchem Isobuten gemeinsam mit 1-Buten und Isobutan über Kopf abgetrennt wird und 2-Butene sowie n-Butan incl. Restmengen an Iso- und 1-Buten im Sumpf verbleiben, oder extraktiv durch Umsetzung von Isobuten mit Alkoholen an sauren Ionenaustauschern. Hierzu werden vorzugsweise Methanol (→ MTBE) oder Isobutanol (IBTBE) eingesetzt.

Die Herstellung von MTBE aus Methanol und Isobuten erfolgt bei 30 bis 100°C und leichtem Überdruck in der Flüssigphase an sauren Ionenaustauschern. Man arbeitet entweder in zwei Reaktoren oder in einem zweistufigen Schachtreaktor, um einen nahezu vollständigen Isobuten-Umsatz (> 99 %) zu erzielen. Die druckabhängige Azeotropbildung zwischen Methanol und MTBE erfordert zur Reindarstellung von MTBE eine mehrstufige Druckdestillation oder wird nach neuerer Technologie durch Methanol-Adsorption an Adsorberharzen erreicht. Alle anderen Komponenten der C₄-Fraktion bleiben unverändert. Da geringe Anteile von Diolefinen und Acetylenen durch Polymerbildung eine Verkürzung der Lebensdauer des Ionenaustauschers bewirken können, werden vorzugsweise bifunktionelle PD-enthaltende Ionenaustauscher eingesetzt, bei denen in Gegenwart kleiner Mengen Wasserstoff nur Diolefine und Acetylene hydriert werden. Die Veretherung des Isobutens bleibt hiervon unbeeinflußt.

MTBE dient in erster Linie zur Octanzahl-Erhöhung von Fahrbenzin. MTBE und IBTBE können alternativ an sauren Oxiden in der Gasphase bei 150 bis 300°C zur Reingewinnung von Isobuten rückgespalten werden.

Eine weitere Möglichkeit zur Abtrennung von Isobuten aus Raffinat I besteht in der direkten Synthese von Oligo/Polyisobuten. An sauren homogenen und heterogen Katalysatoren, wie z.B. Wolframtrioxid auf Titandioxid, kann auf diese Weise bei Isobuten-Umsätzen bis 95 % ein Austragsstrom erhalten werden, der über einen Restanteil an Isobuten von maximal 5 % verfügt.

### Feedreinigung des Raffinat II-Stroms an Adsorbermaterialien

Zur Verbesserung der Standzeit der eingesetzten Katalysatoren für den nachfolgenden Metatheseschritt ist wie vorstehend beschrieben, der Einsatz einer Feed-Reinigung (guard bed) zur Abtrennung von Katalysatorgiften, wie beispielsweise Wasser, Oxygenates, Schwefel oder Schwefelverbindungen bzw. organischen Halogeniden erforderlich.

Verfahren zur Adsorption und adsorptiven Reinigung sind beispielsweise beschrieben in W. Kast, Adsorption aus der Gasphase, VCH, Weinheim (1988). Der Einsatz von zeolithischen Adsorbentien wird erläutert bei D.W. Breck, Zeolite Molecular Sieves, Wiley, New York (1974).

Die Entfernung von speziell Acetaldehyd aus C₃ bis C₁₅-Kohlenwasserstoffen in flüssiger Phase kann gemäß EP-A-0 582 901 erfolgen.

### Selektivhydrierung von Roh-C₄-Schnitt

Aus der aus einem Steamcracker oder einer Raffinerie stammenden Roh-C₄-Fraktion wird zunächst Butadien (1,2- und 1,3-Butadien) sowie im C₄-Schnitt enthaltene Alkine oder Alkenine in einem zweistufigen Verfahren selektivhydriert. Der aus der Raffinerie stammende C₄-Strom kann gemäß einer Ausführungsform auch direkt in den zweiten Schritt der Selektivhydrierung eingespeist werden.

Der erste Schritt der Hydrierung wird vorzugsweise an einem Katalysator durchgeführt, der 0,1 bis 0,5 Gew.-% Palladium auf Aluminiumoxid als Träger enthält. Die Umsetzung wird in Gas/Flüssigphase im Festbett (Rieselfahrweise) mit einem Flüssigkreislauf betrieben. Die Hydrierung erfolgt bei einer Temperatur im Bereich 40 bis 80°C und einem Druck von 10 bis 30 bar, einem Molverhältnis von Wasserstoff zu Butadien von 10 bis 50 und einer Volumengeschwindigkeit LHSV von bis 15 m³ Frischfeed pro m³ Katalysator pro Stunde und einem Verhältnis von Recycle von Zustrom von 5 bis 20 betrieben.

Der zweite Schritt der Hydrierung wird vorzugsweise an einem Katalysator durchgeführt, der 0,1 bis 0,5 Gew.-% Palladium auf Aluminiumoxid als Träger enthält. Die Umsetzung wird in Gas/Flüssigphase im Festbett (Rieselfahrweise) mit einem Flüssigkreislauf betrieben. Die Hydrierung erfolgt bei einer Temperatur im Bereich von 50 bis 90°C und einem Druck von 10 bis 30 bar, einem Molverhältnis von Wasserstoff zu Butadien von 1,0 bis 10 und einer Volumengeschwindigkeit LHSV von 5 bis 20 m³ Frischfeed pro m³ Katalysator pro Stunde und einem Verhältnis von Recycle zu Zustrom von 0 bis 15 betrieben.

Der so erhaltene Reaktionsaustrag wird als Raffinat I bezeichnet und weist neben Isobuten 1-Buten und 2-Buten in einem Molverhältnis von 2:1 bis 1:10, vorzugsweise von 2:1 bis 1:3 auf.

### Alternativ: Abtrennung von Butadien aus Roh-C₄-Schnitt via Extraktion

Die Extraktion von Butadien aus Roh-C₄-Schnitt erfolgt nach BASF-Technologie unter Verwendung von N-Methylpyrrolidon.
Der Reaktionsaustrag der Extraktion wird gemäß einer Ausführungsform der Erfindung in den zweiten Schritt der vorangehend beschriebenen Selektivhydrierung eingespeist, um Restmengen Butadien zu entfernen, wobei in diesem Selektivhydrierungsschritt das gewünschte Verhältnis 1-Buten zu 2-Buten eingestellt wird.

### Abtrennung von Isobuten via Veretherung mit Alkoholen

In der Veretherungsstufe wird Isobuten mit Alkoholen, vorzugsweise mit Isobutanol, an einem sauren Katalysator, vorzugsweise an einem sauren Ionenaustauscher, zu Ether, vorzugsweise Isobutyl-tert.-butylether umgesetzt. Die Umsetzung erfolgt gemäß einer Ausführungsform der Erfindung in einer dreistufigen Reaktorkaskade, in der geflutete Festbettkatalysatoren von oben nach unten durchströmt werden. Im ersten Reaktor beträgt die Eingangstemperatur 0 bis 60°C, vorzugsweise 10 bis 50°C; die Ausgangstemperatur liegt zwischen 25 und 85°C, vorzugsweise zwischen 35 und 75°C, und der Druck beträgt 2 bis 50 bar, vorzugsweise 3 bis 20 bar. Bei einem Verhältnis von Isobutanol zu Isobuten von 0,8 bis 2,0, vorzugsweise 1,0 bis 1,5 beträgt der Umsatz zwischen 70 und 90 %.

Im zweiten Reaktor beträgt die Eingangstemperatur 0 bis 60°C, vorzugsweise 10 bis 50°C; die Ausgangstemperatur liegt zwischen 25 und 85, vorzugsweise zwischen 35 und 75°C, und der Druck beträgt 2 bis 50 bar, vorzugsweise 3 bis 20 bar. Der Gesamtumsatz über die zwei Stufen erhöht sich auf 85 bis 99 %, vorzugsweise 90 bis 97 %.

Im dritten und größten Reaktor wird bei gleicher Eingangs- und Ausgangstemperatur von 0 bis 60°C, vorzugsweise 10 bis 50°C; der Gleichgewichtsumsatz erzielt. An die Veretherung und Abtrennung des gebildeten Ethers schließt sich die Etherspaltung an: Die endotherme Reaktion wird an sauren Katalysatoren, vorzugsweise an sauren Heterogenkontakten, beispielsweise Phosphorsäure auf einem SiO₂-Träger, bei einer Eingangstemperatur von 150 bis 300°C, vorzugsweise bei 200 bis 250°C, und einer Ausgangstemperatur von 100 bis 250°C, vorzugsweise bei 130 bis 220°C durchgeführt.

Bei Einsatz von FCC-C₄-Schnitt ist damit zu rechnen, daß Propan in Mengen um 1 Gew.-%, Isobuten in Mengen um 30 bis 40 Gew.-% sowie C₅-Kohlenwasserstoffe in Mengen um 3 bis 10 % eingeschleust werden, welche die nachfolgende Verfahrenssequenz beeinträchtigen können. Im Rahmen der Aufarbeitung des Ethers ist demzufolge die Möglichkeit einer destillativen Abtrennung der genannten Komponenten vorgesehen.

Der so erhaltene, als Raffinat II bezeichnete Reaktionsaustrag weist einen Isobuten-Restgehalt von 0,1 bis 3 Gew.-% auf.

Bei größeren Mengen an Isobuten im Austrag, wie beispielsweise bei Einsatz von FCC-C₄-Fraktionen oder bei der Abtrennung von Isobuten durch sauerkatalysierte Polymerisation zu Polyisobuten (Teilumsatz), kann der verbleibende Raffinatstrom gemäß einer Ausführungsform der Erfindung vor der Weiterverarbeitung destillativ aufbereitet werden.

### Reinigung des Raffinat II-Stroms an Adsorbermaterialien

Der nach der Veretherung/Polymerisation (bzw. Destillation) erhaltene Raffinat II-Strom wird an mindestens einem guard bed, bestehend aus hochoberflächigen Aluminiumoxiden, Kieselgelen, Alumsolikaten oder Molsieben, gereinigt. Das Schutzbett dient hierbei zum Trocknen des C₄-Stroms sowie zur Entfernung von Substanzen, welche als Katalysatorgift im nachfolgenden Metatheseschritt wirken können. Die bevorzugten Adsorbermaterialien sind Selexsorb CD und CDO sowie 3Å- und NaX-Molsiebe (13X). Die Reinigung erfolgt in Trockentürmen bei Temperaturen und Drucken, die so gewählt sind, daß sämtliche Komponenten in der flüssigen Phase vorliegen. Gegebenenfalls wird der Reinigungsschritt zur Feed-Vorwärmung für den nachfolgenden Metatheseschritt eingesetzt.

Der verbleibende Raffinat II-Strom ist annähernd frei von Wasser, Oxygenaten, organischen Chloriden und Schwefelverbindungen.

Bei Durchführung des Veretherungsschritts mit Methanol zur Herstellung von MTBE kann es aufgrund der Bildung von Dimethylether als Nebenkomponente erforderlich sein, mehrere Reinigungsschritte zu kombinieren bzw. hintereinander zu schalten.

Zur Maximierung der Ausbeute an Propen und 3-Hexen werden im erfindungsgemäßen Verfahren folgende Varianten, welche nachfolgend in der Zeichnung in Fig. 1 und Fig. 2 anhand vereinfachter Verfahrensschemata erläutert werden, bevorzugt. Die Umsetzungen sind zur besseren Übersicht jeweils ohne signifikante Mengen Isobuten im C₄-Feed dargestellt. Dabei bedeuten:
- Et: = Ethen
- C₂⁼: = Ethen
- C₃⁼: = Propen
- C₄⁼: = 1- und 2-Buten
- C₄⁻: = n- und i-Butan
- C₅⁼: = 2-Penten
- C₆⁼: = 3-Hexen
- C₄-Re: = C₄-Recycle
- n-Bu: = n-Butene
- C₂-Re: = C₂-Recycle
- C₅-Re: = C₅-Recycle

Das in der Fig. 1 vereinfacht dargestellte Verfahrensschema beinhaltet eine Reaktoreinheit, die nach herkömmlichem Stand der Technik bevorzugt aus zwei im Swingmodus Synthese/Regeneration betriebenen Reaktoren R besteht, sowie einer dreistufigen Destillationssequenz, die neben dem C₄-Purgestrom die Ausschleusung reiner C₃-, C₅- und/oder C₆-Olefinströme ermöglicht.

Frisch-Raffinat II gelangt zusammen mit Frisch-Ethylen sowie den Recycleströmen C₂⁼, C₄⁻/C₄⁼ und gegebenenfalls C₅⁼ in den bevorzugt als Festbett betriebenen Reaktor R. Dessen aus C₂-C₆-Olefinen und Butanen bestehender Austragsstrom wird in der Destillation D1 getrennt in eine aus Ethylen und Propylen bestehende Leichtsiederfraktion, welche entweder in die Aufarbeitungssequenz eines Crackers eingespeist werden kann oder vorzugsweise in einer weiteren Destillationskolonne D3 in die Reinkomponenten Ethylen und Propylen getrennt wird, sowie in eine Hochsiederfraktion, die aus C₄-Olefinen und Butanen sowie gebildetem 2-Penten und 3-Hexen besteht. In D3 über Kopf abgezogenes Ethylen wird zumindest teilweise in den Metathesereaktor zurückgeführt. Der Sumpfabzug von Kolonne D1 wird in einer weiteren Kolonne D2, die gegebenenfalls als Seitenabzugskolonne oder Trennwandkolonne ausgelegt sein kann, getrennt in eine aus C₄-Olefinen und Butanen bestehende Leichtsiederfraktion, die ganz oder teilweise in den Metatheseschritt zurückgeführt werden kann, in eine vorzugsweise aus 2-Penten bestehende Mittelsiederfraktion, die ganz oder teilweise in den Metatheseschritt zurückgeführt werden kann, und in eine aus 3-Hexen in hoher Reinheit von mindestens 99 % bestehende Wertproduktfraktion, die vorzugsweise ausgeschleust wird.

In einer speziellen Ausführungsform des erfindungsgemäßen Verfahrens ist vorgesehen, die Reaktoreinheit R und die Destillationskolonne D1 zu einer Reaktivdestillationseinheit zu verknüpfen.

Das in der Fig. 2 vereinfacht dargestellte Verfahrensschema beinhaltet eine Reaktoreinheit, die nach herkömmlichem Stand der Technik bevorzugt aus zwei im Swingmodus Synthese/Regeneration betriebenen Reaktoren R besteht, sowie einer vierstufigen Destillationssequenz, die neben dem C₄-Purgestrom die Ausschleusung reiner C₃-, C₅- und/oder C₆-Olefinströme ermöglicht.

Frisch-Raffinat II gelangt zusammen mit Frisch-Ethylen sowie den Recycleströmen C₂⁼, C₄⁻/C₄⁼ und gegebenenfalls C₅⁼ in den bevorzugt als Festbett betriebenen Reaktor R. Dessen aus C₂-C₆-Olefinen und Butanen bestehender Austragsstrom wird in der Destillation D1 getrennt in eine aus Ethylen und Propylen bestehende Leichtsiederfraktion, welche entweder in die Aufarbeitungssequenz eines Crackers eingespeist werden kann oder vorzugsweise in einer weiteren Destillationskolonne D4 in die Reinkomponenten Ethylen und Propylen getrennt wird, sowie in eine Hochsiederfraktion, die aus C₄-Olefinen und Butanen sowie gebildetem 2-Penten und 3-Hexen besteht. In D4 über Kopf abgezogenes Ethylen wird zumindest teilweise in den Metathesereaktor zurückgeführt. Der Sumpfabzug von Kolonne D1 wird in einer weiteren Kolonne D2 getrennt in eine aus C₄-Olefinen und Butanen bestehende Leichtsiederfraktion, die ganz oder teilweise in den Metatheseschritt zurückgeführt werden kann sowie in eine aus 2-Penten und 3-Hexen bestehende Hochsiederfraktion, die in der Kolonne D3 getrennt wird in die Reinkomponenten 2-Penten als Kopfabzug, welcher ganz oder zumindest teilweise in den Metathesereaktor zurückgeführt werden kann, sowie eine mindestens 99 % Hexene enthaltende C₆-Wertproduktfraktion, die ausgeschleust wird.

Als Katalysatoren werden literaturbekannte heterogene Rhenium-Katalysatoren, wie Re₂O₇ auf γ-Al₂O₃ oder auf Mischträgem, wie z.B. SiO₂/Al₂O₃, B₂O₃/SiO₂/Al₂O₃ oder Fe₂O₃/Al₂O₃ mit unterschiedlichem Metallgehalt bevorzugt. Der Rheniumoxid-Gehalt beträgt unabhängig vom gewählten Träger zwischen 1 und 20 %, vorzugsweise zwischen 3 und 10%.
Die Katalysatoren werden frisch calziniert eingesetzt und bedürfen keiner weiteren Aktivierung (z.B. durch Alkylierungsmittel). Desaktivierter Katalysator kann durch Abbrennen von Coke-Rückständen bei Temperaturen oberhalb von 400°C im Luftstrom und Abkühlung unter Inertgas-Atmosphäre mehrfach regeneriert werden.

Ein Vergleich der Heterogenkontakte untereinander zeigt, daß Re₂O₇/Al₂O₃ bereits unter sehr milden Reaktionsbedingungen (T = 20 bis 80°C) aktiv ist, während MO₃/SiO₂ (M = Mo, W) erst bei Temperaturen oberhalb von 100 bis 150°C Aktivität entwickelt und demzufolge als Nebenreaktionen C=C-Doppelbindungsisomerisierung auftreten kann.

Ferner sind zu nennen:
- WO₃/SiO₂, präpariert aus (C₅H₅)W(CO)₃Cl und SiO₂ in J. Mol Catal. 1995, 95, 75-83;
- 3-Komponenten-System, bestehend aus [Mo(NO)₂(OR)₂]n, SnEt₄ und AlCl₃ in J. Mol. Catal. 1991, 64, 171-178 und J. Mol. Catal 1989, 57, 207-220;
- Nitridomolybdän (VI)-Komplexe aus hochaktive Präkatalysatoren in J. Organomet. Chem. 1982, 229, C19-C23;
- heterogene SiO₂-geträgerte MoO₃ und WO₃-Katalysatoren in J. Chem. Soc., Faraday Trans. /1982, 78, 2583-2592;
- geträgerte Mo-Katalysatoren in J. Chem. Soc., Faraday Trans. / 1981, 77,1763-1777;
- aktive Wolfram-Katalysatorvorstufe in J. Am. Chem. Soc. 1980, 102(21), 6572-6574;
- Acetonitril(pentacarbonyl)wolfram in J. Catal. 1975, 38, 482-484;
- Trichloro(nitrosyl)molybdän(II) als Katalysator-Vorstufe in Z. Chem. 1974, 14, 284-285;
- W(CO)₅PPH₃/EtAlCl₂ in J. Catal. 1974, 34, 196-202;
- WCl₆/n-BuLi in J. Catal 1973, 28, 300-303;
- WCl₆/n-BuLi in J. Catal. 1972, 26, 455-458;

FR 2 726 563: O₃ReO[Al(OR)(L)xO]nReO₃ mit R = C₁-C₄₀-Kohlenwasserstoff, n = 1-10, x = 0 oder 1 und L = Solvens,
EP-A-191 0 675, EP-A-129 0 474, BE 899897: Katalysatorsysteme aus Wolfram, 2 substituierten Phenolatresten und 4 anderen Liganden, u.a. einer Halogen-, Alkyl- bzw. Carbengruppe.

FR 2 499 083: Katalysatorsystem aus einem Wolfram-, Molybdän- oder Rhenium-Oxo-Übergangsmetallkomplex mit einer Lewissäure.

US 4,060,468: Katalysatorsystem aus einem Wolframsalz, einer sauerstoffhaltigen aromatischen Verbindung, z.B. 2,6-Dichlorphenol und wahlweise molekularem Sauerstoff.

BE 776,564: Katalysatorsystem aus einem Übergangsmetallsalz, einer metallorganischen Verbindung und einem Amin.

Für die Verbesserung der Cyclusdauer der eingesetzten Katalysatoren, vor allem der geträgerten Katalysatoren, empfiehlt sich der Einsatz einer Feed-Reinigung an Adsorberbetten (guard beds). Das Schutzbett dient hierbei zum Trocknen des C₄-Stroms sowie zur Entfernung von Substanzen, welches als Katalysatorgift im nachfolgenden Metatheseschritt wirken können. Die bevorzugten Adsorbermaterialien sind Selexsorb CD und CDO sowie 3Å- und NaX-Molsiebe (13X). Die Reinigung erfolgt in Trockentürmen bei Temperaturen und Drucken, die bevorzugt so gewählt sind, daß sämtliche Komponenten in der flüssigen Phase vorliegen. Gegebenenfalls wird der Reinigungsschritt zur Feed-Vorwärmung für den nachfolgenden Metatheseschritt eingesetzt. Es kann von Vorteil sein, mehrere Reinigungsschritte miteinander zu kombinieren bzw. hintereinander zu schalten.

Druck und Temperatur im Metatheseschritt sind so gewählt, daß sämtliche Reaktionspartner in der flüssigen Phase vorliegen (üblicherweise = 0 bis 150°C, bevorzugt 20 bis 80°C; p = 2 bis 200 bar). Alternativ kann es aber von Vorteil sein, insbesondere bei Feedströmen mit höherem Isobutengehalt, die Umsetzung in der Gasphase durchzuführen und/oder einen Katalysator einzusetzen, der über eine geringere Acidität verfügt.

In der Regel ist die Umsetzung nach 1 s bis 1 h, vorzugsweise nach 30 s bis 30 min beendet. Sie kann kontinuierlich oder diskontinuierlich in Reaktoren, wie Druckgasgefäßen, Strömungsrohren oder Reaktivdestillationsvorrichtungen durchgeführt werden, wobei Strömungsrohre bevorzugt werden.

### Beispiele

### Beispiel 1

### Kontinuierlicher Versuch zur zweistufigen Selektivhydrierung von Roh-C₄-Schnitt

### 1. Stufe

Roh-C₄-Schnitt mit einer Zusammensetzung von 43,7 % Butadien (incl. Butenin und Butin), 14,3 % 1-Buten, 7,8 % 2-Butene und 7,2 % n-Butan wird in einem kontinuierlich durchströmten Rohrreaktor an 0,3 % Pd/Al₂O₃-Heterogenkontakt bei einem Fischzulauf von 1 kg/h Roh-C₄-Schnitt und einem Kreislauf von 8,2 kg/h mit einer LHSV von 9,0 h⁻¹ bei einer Reaktoreingangstemperatur von 20°C mit 175 Nl/h Wasserstoff umgesetzt. Bei einem Butadien-Umsatz von 95,2 % wurde in der ersten Stufe der Selektivhydrierung unter diesen Bedingungen eine Gesamt-Buten-Selektivität von 99,6 % sowie eine 1-Buten-Selektivität von 56,5 % erzielt.

### 2. Stufe

Ein typischer Reaktionsaustrag aus der ersten Stufe der Selektivhydrierung, bestehend aus 0,53 % Butadien (incl. Butenin und Butin), 27,09 % 1-Buten, 14,6 % 2-Butene und 11,0 % n-Butan wird in einem kontinuierlich durchströmten Rohrreaktor an 0,3 % Pd/Al₂O₃-Heterogenkontakt (H0-13L) bei einem Fischzulauf von 1,7 kg/h Reaktionsaustrag der 1. Stufe und einem Kreislauf von 3,0 kg/h mit einer LHSV von 15 h⁻¹ bei einer Reaktoreingangstemperatur von 60°C und einer Reaktorausgangstemperatur von 70°C bzw. 67°C mit 20 Nl/h bzw. 10 Nl/h Wasserstoff umgesetzt. Bei einem Butadien-Umsatz von 99,8 % bzw. 98,5 % wurde unter diesen Bedingungen ein Raffinat I-Strom erhalten, der einen Restgehalt von 10 ppm bzw. 8 ppm Butadien aufweist bei einer n-Butan-Bildung von 2,7 % bzw. 1,3 % und ein Isomerenverhältnis von 2-Buten zu 1-Buten von 3,4 bzw. 1,5 aufweist.

### Beispiel 2

### Kontinuierlicher Versuch zur Abtrennung von Isobuten durch Veretherung mit Isobutanol

In einer dreistufigen Reaktorkaskade wird ein geflutetes und mit saurem Ionenaustauscher bestücktes Festbett von oben nach unten mit Raffinat I und Isobutanol durchströmt, wobei das Verhältnis von Isobutanol zu Isobuten im Feed auf 1,2 eingestellt wurde. Die Reaktoreingangstemperatur liegt bei 40°C, die Reaktorausgangstemperatur bei 65°C und der Reaktionsdruck bei 8 bar. Der gemessene Isobuten-Umsatz nach der ersten Stufe liegt bei 85 %. Im zweiten, ähnlich dimensionierten Reaktor wird der Umsatz bei einer Reaktoreingangstemperatur von 40°C, einer Reaktorausgangstemperatur bei 50°C und einem Reaktionsdruck von 8 bar auf 95 % erhöht. Im dritten, deutlich größeren Reaktor wird der Gleichgewichtsumsatz bei einer Reaktoreingangstemperatur und Reaktorausgangstemperatur von jeweils 40°C und einem Reaktionsdruck von 8 bar eingestellt. Der unter diesen Bedingungen nach destillativer Abtrennung von Isobutyl-tert.-butylether verbleibende Raffinat-Strom weist einen Isobuten-Restgehalt von 0,7 % auf.

### Beispiel 3

### a) Kontinuierlicher Versuch zur einstufigen Metathese von Raffinat II mit Zielrichtung Propylen-Optimierung unter Zudosierung von Ethylen

Nach Feedreinigung über ein Adsorberbett aus Molsieb 13X wird mit einem Massenstrom von 10,44 kg/h eine aus 18,9 % 1-Buten, 66,1 % 2-Buten und 15,0 % Butanen bestehende C₄-Fraktion zusammen mit 2,75 kg/h Ethylen bei 50°C und 30 bar in der Flüssigphase kontinuierlich über einen mit Re₂O₇/Al₂O₃-Heterogenkontakt bestückten Rohrreaktor geleitet. Der Reaktionsaustrag wird nach einer Verweilzeit von 5 min in einer dreistufigen Destillationssequenz getrennt, wobei in der ersten Kolonne eine C₂/C₃-Leichtsiederphase über Kopf genommen wird, welche in einer zweiten Destillationskolonne feindestilliert wird. Hierbei werden 9,62 kg/h PG-Propylen erhalten. Die dabei als Kopfabzug anfallende Ethylenfraktion wird komplett in den Metatheseschritt zurückgeführt. Der aus C₄-C₆-Olefinen und Butanen bestehende Sumpfaustrag der ersten Kolonne wird einer dritten Kolonne zugeführt, in welcher die über Kopf abgetrennte C₄-Leichtsiederfraktion größtenteils in die Metathesereaktion zurückgeführt wird. Eine als Mittelsiederfraktion abgezogene C₅-Olefinreaktion, bestehend aus 98,5 % cis/trans-2-Penten wird größtenteils in den Metathesereaktor zurückgeführt. Die im Sumpf dieser Kolonne anfallende Hochsiederfraktion von 1,38 kg/h bestand zu 99,5 % aus cis/trans-3-Hexen. Der ermittelte Gesamtbutenumsatz lag bei einem C₄-Purgestrom von 2,19 kg/h bei 93,1 %, der Ethylen-Umsatz bei >99 %.

### b) Kontinuierlicher Versuch zur einstufigen Metathese von Raffinat II zu Propylen und 3-Hexen bei vermindertem Ethylen-Einsatz

Nach Feedreinigung über ein Adsorberbett aus Molsieb 13X wird mit einem Massenstrom von 10,44 kg/h eine aus 37,8 kg/h 1-Buten, 47,2 % 2-Buten und 15,0 % Butanen bestehende C₄-Fraktion zusammen mit 1,38 kg/h Ethylen bei 50°C und 25 bar in der Flüssigphase kontinuierlich über einen mit Re₂O₇/Al₂O₃-Heterogenkontakt bestückten Rohrreaktor geleitet. Der Reaktionsaustrag wird nach einer Verweilzeit von 5 min in einer dreistufigen Destillationssequenz getrennt, wobei in der ersten Kolonne eine C₂/C₃-Leichtsiederphase über Kopf genommen wird, welche in einer zweiten Destillationskolonne feindestilliert wird. Hierbei werden 6,88 kg/h PG-Propylen erhalten. Die dabei als Kopfabzug anfallende Ethylenfraktion wird komplett in den Metatheseschritt zurückgeführt. Der aus C₄-C₆-Olefinen und Butanen bestehende Sumpfaustrag der ersten Kolonne wird einer dritten Kolonne zugeführt, in welcher die über Kopf abgetrennte C₄-Leichtsiederfraktion größtenteils in die Metathesereaktion zurückgeführt wird. Eine als Mittelsiederfraktion abgezogene C₅-Olefinfraktion, bestehend aus 98,5 % cis/trans-2-Penten wird größtenteils in den Metathesereaktor zurückgeführt. Die im Sumpf dieser Kolonne anfallende Hochsiederfraktion von 2,75 kg/h bestand zu 99,6 % aus cis/trans-3-Hexen.
Der ermittelte Gesamtbutenumsatz lag bei einem C₄-Purgestrom von 2,20 kg/h bei 93,0 %, der Ethylen-Umsatz bei >99 %.

## Patentansprüche

1. Verfahren zur Herstellung von Propen und Hexen aus einem olefinische C₄-Kohlenwasserstoffe enthaltenden Raffinat-II-Ausgangsstrom, **dadurch gekennzeichnet, daß**
a) in Gegenwart eines Metathesekatalysators, der mindestens eine Verbindung eines Metalls der VI.b, VII.b oder VIII. Nebengruppe des Periodensystems der Elemente enthält, eine Metathesereaktion durchgeführt wird, im Rahmen derer im Ausgangsstrom enthaltene Butene mit Ethen zu einem Ethen, Propen, Butene, 2-Penten, 3-Hexen und Butane enthaltenden Gemisch umgesetzt werden, wobei bezogen auf die Butene 0,05 bis 0,6 Moläquivalente Ethen eingesetzt werden,
b) der so erhaltene Austragsstrom zunächst destillativ getrennt wird in eine C₂-C₃-Olefine enthaltende Leichtsiederfraktion **A** sowie in eine C₄-C₆-Olefine und Butane enthaltende Schwersiederfraktion,
c) die aus b) erhaltene Leichtsiederfraktion **A** anschließend in die Aufarbeitungssequenz eines Crackers eingespeist wird oder destillativ in eine Ethen enthaltende Fraktion und eine Propen enthaltende Fraktion getrennt wird, wobei die Ethen enthaltende Fraktion in den Verfahrensschritt a) zurückgeführt wird und die Propen enthaltende Fraktion als Produkt ausgeschleust wird,
d) die aus b) erhaltene Schwersiederfraktion anschließend destillativ in eine Butene und Butane enthaltende Leichtsiederfraktion **B**, eine Penten enthaltende Mittelsiederfraktion **C** und in eine Hexen enthaltende Schwersiederfraktion **D** getrennt wird,
e) wobei die Fraktionen **B** und **C** komplett oder teilweise in den Verfahrensschritt a) zurückgeführt werden und die Fraktion **D** als Produkt ausgeschleust wird.

2. Verfahren nach Anspruch 1, **dadurch gekennzeichnet, daß** Schritt d) in einer Trennwandkolonne durchgeführt wird.

3. Verfahren nach Anspruch 1, **dadurch gekennzeichnet, daß** in Schritt d) die aus b) erhaltene Schwersiederfraktion zunächst destillativ in eine Butene und Butane enthaltende Leichtsiederfraktion **B** und eine 2-Penten und 3-Hexen enthaltende Hochsiederfraktion getrennt wird, wobei die Hochsiederfraktion sodann destillativ in die Fraktionen **C** und **D** getrennt wird.

4. Verfahren nach einem der Ansprüche 1 bis 3, **dadurch gekennzeichnet, daß** die Metathesereaktion in Gegenwart von heterogenen Metathesekatalysatoren durchgeführt wird, die aus der Klasse der auf anorganischen Trägern aufgebrachten Übergangsmetallverbindungen von Metallen der VI.b, VII.b oder VIII. Gruppe des Periodensystems der Elemente ausgewählt sind.

5. Verfahren nach Anspruch 4, **dadurch gekennzeichnet, daß** als Metathesekatalysator Rheniumoxid auf γ-Aluminiumoxid oder auf Al₂O₃/B₂O₃/SiO₂-Mischträgern eingesetzt wird.

6. Verfahren zur Herstellung von Propen und Hexen aus Steamcracker- oder Raffinerie-C₄-Strömen, umfassend die Teilschritte
(1) Abtrennung von Butadien und acetylenischen Verbindungen durch gegebenenfalls Extraktion von Butadien mit einem Butadien-selektiven Lösungsmittel und nachfolgend /oder Selektivhydrierung von in Roh-C₄-Schnitt enthaltenen Butadienen und acetylenischen Verunreinigungen, um einen Reaktionsaustrag zu erhalten, der n-Butene und Isobuten und im wesentlichen keine Butadiene und acetylenischen Verbindungen enthält,
(2) Abtrennung von Isobuten durch Umsetzung des in der vorstehenden Stufe erhaltenen Reaktionsaustrags mit einem Alkohol in Gegenwart eines sauren Katalysators zu einem Ether, Abtrennung des Ethers und des Alkohols, die gleichzeitig oder nach der Veretherung erfolgen kann, um einen Reaktionsaustrag zu erhalten, der n-Butene und gegebenenfalls Oxygenat-Verunreinigungen enthält, wobei gebildeter Ether ausgetragen oder zur Reingewinnung von Isobuten rückgespalten werden kann und dem Veretherungsschritt ein Destillationsschritt zur Abtrennung von Isobuten nachfolgen kann, wobei gegebenenfalls auch eingeschleuste C₃-,i-C₄- sowie C₅-Kohlenwasserstoffe destillativ im Rahmen der Aufarbeitung des Ethers abgetrennt werden können,
(3) Abtrennen der Oxygenat-Verunreinigungen des Austrags der vorstehenden Schritte an entsprechend ausgewählten Adsorbermaterialien,
(4) Weitere Umsetzung des so erhaltenen Raffinat II-Stromes in einem Verfahren gemäß einem oder mehreren der Ansprüche 1 bis 5.

7. Verfahren nach Anspruch 6, **dadurch gekennzeichnet, daß** der Teilschritt Selektivhydrierung von in Roh-C₄-Schnitt enthaltenen Butadienen und acetylenischen Verunreinigungen zweistufig durchgeführt wird durch Inkontaktbringen des Roh-C₄-Schnittes in flüssiger Phase mit einem Katalysator, der mindestens ein Metall, ausgewählt aus der Gruppe Nickel, Palladium und Platin, auf einem Träger enthält, bei einer Temperatur von 20 bis 200°C, einem Druck von 1 bis 50 bar, einer Volumengeschwindigkeit von 0,5 bis 30 m³ Frischfeed pro m³ Katalysator pro Stunde und einem Verhältnis von Recycle zu Zustrom von 0 bis 30 mit einem Molverhältnis von Wasserstoff zu Diolefinen von 0,5 bis 50, um einen Reaktionsaustrag zu erhalten, in welchem neben Isobuten die n-Butene 1-Buten und 2-Buten in einem Molverhältnis von 2:1 bis 1:10 vorliegen und im wesentlichen keine Diolefine und acetylenischen Verbindungen enthalten sind.

8. Verfahren nach Anspruch 6 oder 7, **dadurch gekennzeichnet, daß** der Teilschritt Butadien-Extraktion aus Roh-C₄-Schnitt mit einem Butadien-selektiven Lösungsmittel ausgewählt aus Aceton, Furfural, Acetonitril, Dimethylacetamid, Dimethylformamid und N-Methylpyrrolidon, durchgeführt wird, um einen Reaktionsaustrag zu erhalten, in welchem nach anschließend durchgeführter Selektivhydrierung/Isomerisierung die n-Butene 1-Buten und 2-Buten in einem Molverhältnis von 2:1 bis 1:10, vorzugsweise von 2:1 bis 1:3 vorliegen.

9. Verfahren nach einem der Ansprüche 6 bis 8, **dadurch gekennzeichnet, daß** der Teilschritt Isobuten-Veretherung mit Methanol oder Isobutanol in Gegenwart eines sauren Ionenaustauschers in einer dreistufigen Reaktorkaskade durchgeführt wird, in der geflutete Festbettkatalysatoren von oben nach unten durchströmt werden, wobei die Reaktor-Eingangstemperatur 0 bis 60°C, die Ausgangstemperatur 25 bis 85°C, der Druck 2 bis 50 bar und das Verhältnis von Isobutanol zu Isobuten 0,8 bis 2,0 betragen sowie der Gesamtumsatz dem Gleichgewichtsumsatz entspricht.

10. Verfahren nach einem der Ansprüche 6 bis 8, **dadurch gekennzeichnet, daß** der Teilschritt Isobuten-Abtrennung durch Oligomerisierung oder Polymerisation von Isobuten ausgehend von dem Reaktionsaustrag, der nach den vorstehend beschriebenen Stufen Butadien-Extraktion und/oder Selektivhydrierung erhalten wird, in Gegenwart eines Katalysators verläuft, welcher ausgewählt ist aus heterogenen Kontakten, die ein Oxid eines Metalls der VI.b Nebengruppe des Periodensystems der Elemente auf einem sauren anorganischen Träger enthalten, um so einen Strom zu erzeugen, welcher weniger als 15 % Isobuten-Restgehalt aufweist.

11. Verfahren nach einem der Ansprüche 6 bis 10, **dadurch gekennzeichnet, daß** der Teilschritt Feedreinigung an mindestens einem Guard Bed, bestehend aus hochoberflächigen Aluminiumoxiden, Kieselgelen, Alumosilikaten oder Molsieben, durchgeführt wird.

## Claims

1. A process for preparing propene and hexene from a raffinate II feed stream comprising olefinic C₄-hydrocarbons, which comprises
a) a metathesis reaction in which butenes present in the feed stream are reacted with ethene in the presence of a metathesis catalyst comprising at least one compound of a metal of transition group VIb, VIIb or VIII of the Periodic Table of the Elements to give a mixture comprising ethene, propene, butenes, 2-pentene, 3-hexene and butanes, using, based on the butenes, from 0.05 to 0.6 molar equivalents of ethene,
b) firstly fractionally distilling the product stream obtained in this way to give a low-boiling fraction A comprising C₂-C₃-olefins and a high-boiling fraction comprising C₄-C₆-olefins and butanes,
c) subsequently feeding the low-boiling fraction A obtained from b) into the processing sequence of a cracker or fractionally distilling said fraction A to give an ethene-comprising fraction and a propene-comprising fraction, with the ethene-comprising fraction being returned to the process step a) and the propene-comprising fraction being discharged as product,
d) subsequently fractionally distilling the high-boiling fraction obtained from b) to give a low-boiling fraction B comprising butenes and butanes, a pentene-comprising intermediate-boiling fraction C and a hexene-comprising high-boiling fraction D,
e) where the fractions B and C are completely or partly returned to the process step a) and the fraction D is discharged as product.

2. The process according to Claim 1, wherein step d) is carried out in a dividing wall column.

3. The process according to Claim 1, wherein, in step d), the high-boiling fraction obtained from b) is firstly fractionally distilled to give a low-boiling fraction B comprising butenes and butanes and a high-boiling fraction comprising 2-pentene and 3-hexene, and the high-boiling fraction is then fractionally distilled to give the fractions C and D.

4. The process according to any of Claims 1 to 3, wherein the metathesis reaction is carried out in the presence of heterogeneous metathesis catalysts which are selected from the group consisting of transition metal compounds of metals of groups VIb, VIIb and VIII of the Periodic Table of the Elements applied to inorganic supports.

5. The process according to Claim 4, wherein the metathesis catalyst used is rhenium oxide on γ-aluminum oxide or on Al₂O₃/B₂O₃/SiO₂ mixed supports.

6. The process for preparing propene and hexene from steam cracker or refinery C₄ streams, which comprises the substeps
(1) removal of butadiene and acetylenic compounds by, if appropriate, extraction of butadiene with a butadiene-selective solvent and subsequent/or selective hydrogenation of butadienes and acetylenic impurities present in crude C₄ fraction to give a reaction product comprising n-butenes and isobutene and essentially no butadienes and acetylenic compounds,
(2) removal of isobutene by reaction of the reaction product obtained in the preceding step with an alcohol in the presence of an acid catalyst to form an ether, removal of the ether and the alcohol, which can be carried out simultaneously with or after the etherification, to give a reaction product comprising n-butenes and possibly oxygen-containing impurities, with the ether formed being able to be discharged or redissociated to obtain pure isobutene and the etherification step being able to be followed by a distillation step for removing isobutene, where, if desired, introduced C₃-, i-C₄- and C₅-hydrocarbons can also be separated off by distillation during the work-up of the ether,
(3) removal of the oxygen-containing impurities from the output from the preceding steps over appropriately selected adsorber materials,
(4) further reaction of the resulting raffinate II stream in a process according to one or more of Claims 1 to 5.

7. The process according to Claim 6, wherein the substep of selective hydrogenation of butadienes and acetylenic impurities present in crude C₄ fraction is carried out in two stages by bringing the crude C₄ fraction in the liquid phase into contact with a catalyst comprising at least one metal selected from the group consisting of nickel, palladium and platinum on a support at from 20 to 200°C, a pressure of from 1 to 50 bar, an LHSV of from 0.5 to 30 m³ of fresh feed per m³ of catalyst per hour and a ratio of recycle to fresh feed of from 0 to 30 and a molar ratio of hydrogen to diolefins of from 0.5 to 50 to give a reaction product comprising, apart from isobutene, the n-butenes 1-butene and 2-butene in a molar ratio of from 2:1 to 1:10 and comprising essentially no diolefins and acetylenic compounds.

8. The process according to Claim 6 or 7, wherein the substep of butadiene extraction from crude C₄ fraction is carried out using a butadiene-selective solvent selected from the group consisting of acetone, furfural, acetonitrile, dimethylacetamide, dimethylformamide and N-methylpyrrolidone to give a reaction product in which, after subsequent selective hydrogenation/isomerization, the n-butenes 1-butene and 2-butene are present in a molar ratio of from 2:1 to 1:10, preferably from 2:1 to 1:3.

9. The process according to any of Claims 6 to 8, wherein the substep of isobutene etherification is carried out using methanol or isobutanol in the presence of an acid ion exchanger in a three-stage reactor cascade, where the reaction mixture flows from the top downward through the flooded fixed-bed catalysts and the reactor inlet temperature is from 0 to 60°C, the outlet temperature is from 25 to 85°C, the pressure is from 2 to 50 bar, the ratio of isobutanol to isobutene is from 0.8 to 2.0 and the total conversion corresponds to the equilibrium conversion.

10. The process according to any of Claims 6 to 8, wherein the substep of isobutene removal is carried out by oligomerization or polymerization of isobutene in the presence of a catalyst selected from the group consisting of heterogeneous catalysts comprising an oxide of a metal of transition group VIb of the Periodic Table of the Elements on an acidic inorganic support, starting from the reaction product obtained after the above-described stages of butadiene extraction and/or selective hydrogenation so as to produce a stream having a residual isobutene content of less than 15%.

11. The process according to any of Claims 6 to 10, wherein the substep of feed purification is carried out using at least one guard bed comprising high surface area aluminum oxides, silica gels, aluminosilicates or molecular sieves.

## Revendications

1. Procédé pour la production de propène et d'hexène à partir d'un courant de sortie de raffinat II contenant des hydrocarbures oléfiniques en C₄, **caractérisé en ce que**
a) on effectue une réaction de métathèse en présence d'un catalyseur de métathèse qui contient au moins un composé d'un métal du groupe VI.b, VII.b ou VIII. du système périodique des éléments, dans le cadre de laquelle on fait réagir avec de l'éthène les butènes contenus dans le courant de sortie, pour obtenir un mélange contenant de l'éthène, du propène, des butènes, du 2-pentène, du 3-hexène et des butanes, en utilisant de 0,05 à 0,6 équivalent molaire d'éthène par rapport aux butènes,
b) le courant de décharge ainsi obtenu est d'abord fractionné par distillation en une fraction contenant des composés à bas point d'ébullition **A**, qui contient des oléfines en C₂-C₃, ainsi qu'en une fraction contenant des composés à haut point d'ébullition, qui contient des oléfines en C₄-C₆ et des butanes,
c) la fraction contenant des composés à bas point d'ébullition **A**, obtenue à partir de b), est ensuite introduite dans la séquence de traitement d'une unité de craquage ou fractionnée par distillation en une fraction contenant de l'éthène et une fraction contenant du propène, la fraction contenant de l'éthène étant renvoyée dans l'étape a) du processus et la fraction contenant du propène étant évacuée en tant que produit,
d) la fraction contenant des composés à haut point d'ébullition, obtenue à partir de b) est ensuite fractionnée par distillation en une fraction contenant des composés à bas point d'ébullition **B,** qui contient des butènes et des butanes, une fraction contenant des composés à point d'ébullition moyen **C,** qui contient du pentène, et une fraction contenant des composés à haut point d'ébullition **D,** qui contient de l'hexène,
e) les fractions **B** et **C** étant renvoyées en totalité ou en partie dans l'étape a) du processus et la fraction **D** étant évacuée en tant que produit.

2. Procédé selon la revendication 1, **caractérisé en ce que** l'étape d) est effectuée dans une colonne à paroi de séparation.

3. Procédé selon la revendication 1, **caractérisé en ce que** dans l'étape d) la fraction contenant des composés à haut point d'ébullition, obtenue à partir de b), est d'abord fractionnée par distillation en une fraction contenant des composés à bas point d'ébullition **B,** qui contient des butènes et des butanes, et une fraction contenant des composés à haut point d'ébullition, qui contient du 2-pentène et du 3-hexène, la fraction contenant des composés à haut point d'ébullition étant ensuite fractionnée par distillation en les fractions **C** et **D.**

4. Procédé selon l'une quelconque des revendications 1 à 3, **caractérisé en ce que** la réaction de métathèse est effectuée en présence de catalyseurs de métathèse hétérogènes, qui sont choisis dans la classe des composés de métaux de transition des métaux du groupe VI.b, VII.b ou VIII. du système périodique des éléments, appliqués sur des supports inorganiques.

5. Procédé selon la revendication 4, **caractérisé en ce qu'**on utilise comme catalyseur de métathèse de l'oxyde de rhénium sur oxyde d'aluminium γ ou sur des supports mixtes Al₂O₃/B₂O₃/SiO₂.

6. Procédé pour la production de propène et d'hexène à partir de courants en C₄ de raffineries ou d'unités de vapocraquage, comprenant les étapes partielles de
(1) séparation du butadiène et de composés acétyléniques par éventuellement extraction du butadiène par un solvant sélectif pour le butadiène et ensuite/ou hydrogénation sélective des butadiènes contenus dans la fraction en C₄ brute et des impuretés acétyléniques, pour l'obtention d'un courant de décharge de réaction qui contient des n-butènes et de l'isobutène et ne contient pratiquement pas de butadiènes ni de composés acétyléniques,
(2) séparation de l'isobutène par réaction de la décharge de réaction obtenue dans l'étape précédente avec un alcool en présence d'un catalyseur acide pour la formation d'un éther, séparation de l'éther et de l'alcool, qui peut s'effectuer en même temps que ou après l'éthérification, pour l'obtention d'un courant de décharge de réaction qui contient des n-butènes et éventuellement des impuretés de type produit d'oxygénation, l'éther formé pouvant être déchargé ou redissocié pour l'obtention d'isobutène pur et à l'étape d'éthérification pouvant faire suite une étape de distillation pour la séparation d'isobutène, les hydrocarbures en C₃, iso-C₄ ainsi qu'en C₅ éventuellement entraînés également pouvant être séparés par distillation dans le cadre du traitement de l'éther,
(3) séparation des impuretés de type produit d'oxygénation de la décharge des étapes précédentes, sur des matières adsorbantes convenablement choisies,
(4) nouvelle réaction du courant de raffinat II ainsi obtenu, dans un procédé selon une ou plusieurs des revendications 1 à 5.

7. Procédé selon la revendication 6, **caractérisé en ce que** l'étape partielle hydrogénation sélective des butadiènes contenus dans la fraction en C₄ brute et des impuretés acétyléniques est effectuée en deux stades par mise en contact de la fraction en C₄ brute en phase liquide avec un catalyseur qui contient au moins un métal, choisi dans le groupe constitué par le nickel, le palladium et le platine, sur un support, à une température de 20 à 200 °C, sous une pression de 1 à 50 bars, à une vitesse volumique de 0,5 à 30 m³ de charge nouvelle par m³ de catalyseur par heure et à un rapport du courant de recyclage au courant d'alimentation de 0 à 30 avec un rapport molaire de l'hydrogène aux dioléfines de 0,5 à 50, pour l'obtention d'un courant de décharge de réaction dans lequel, outre l'isobutène, les n-butènes 1-butène et 2-butène sont présents en un rapport molaire de 2:1 à 1:10 et pratiquement aucune dioléfine ni aucun composé acétylénique ne sont contenus.

8. Procédé selon la revendication 6 ou 7, **caractérisé en ce que** l'étape partielle extraction du butène à partir de la fraction en C₄ brute est effectuée à l'aide d'un solvant sélectif pour le butadiène, choisi parmi l'acétone, le furfural, l'acétonitrile, le diméthylacétamide, le diméthylformamide et la N-méthylpyrrolidone, pour l'obtention d'un courant de décharge de réaction dans lequel, après hydrogénation sélective/isomérisation effectuée ensuite, les n-butènes 1-butène et 2-butène sont présents en un rapport molaire de 2:1 à 1:10, de préférence de 2:1 à 1:3.

9. Procédé selon l'une quelconque des revendications 6 à 8, **caractérisé en ce que** l'étape partielle éthérification d'isobutène par le méthanol ou l'isobutanol est effectuée en présence d'un échangeur d'ions acide dans une cascade de réacteurs à trois étages, dans laquelle des catalyseurs en lit fixe à ruissellement sont traversés de haut en bas, la température d'entrée dans le réacteur étant dans la plage de 0 à 60 °C, la température de sortie étant dans la plage de 25 à 85 °C, la pression étant de 2 à 50 bars et le rapport de l'isobutanol à l'isobutène valant de 0,8 à 2,0 et le degré de conversion global correspondant à la conversion à l'équilibre.

10. Procédé selon l'une quelconque des revendications 6 à 8, **caractérisé en ce que** l'étape partielle séparation d'isobutène par oligomérisation ou polymérisation d'isobutène à partir de la décharge de réaction qui est obtenue après les étapes décrites précédemment d'extraction du butadiène et/ou d'hydrogénation sélective, se déroule en présence d'un catalyseur qui est choisi parmi des catalyseurs hétérogènes qui contiennent un oxyde d'un métal du groupe VI.b du système périodique des éléments, sur un support inorganique acide, pour la production d'un courant qui présente une teneur résiduelle en isobutène inférieure à 15 %.

11. Procédé selon l'une quelconque des revendications 6 à 10, **caractérisé en ce que** l'étape partielle purification de la charge d'alimentation est effectuée sur au moins un Guard Bed, constitué d'oxydes d'aluminium à grande surface spécifique, de gels de silice, d'aluminosilicates ou de tamis moléculaires.
